# EUROPEAN PATENT SPECIFICATION

(11) **EP 2 612 624 B1**
(45) Date of publication and mention of the grant of the patent: **05.11.2014**
(21) Application number: 12150517.6
(22) Date of filing: 09.01.2012
(51) Int. Cl.: A61F 5/01

(54) **Functional knee brace for posterior cruciate ligament support**
Funktionale Kniemanschette für eine posteriore Kreuzbandstützung
Attelle de genou fonctionnelle pour le support postérieur des ligaments croisés

(43) Date of publication of application: 10.07.2013
(73) Proprietor: F.G.P. S.r.l., 37062 Dossobuono (VR) (IT)
(72) Inventor: Turrini, Alberto, 37062 Dossobuono di Villafranca (VR) (IT); Ferrigolo, Moreno, 37062 Dossobuono di Villafranca (VR) (IT)
(74) Representative: Sandri, Sandro

(56) References cited:
- EP-A1- 1 302 184
- US-A- 4 361 142
- US-A- 5 891 061
- US-A1- 2005 273 025

## Description

### TECHNICAL FIELD

This invention concerns a functional knee brace designed to support the posterior cruciate ligament.

More specifically, this invention refers to a functional knee brace designed to support the posterior cruciate ligament, which can be used for rehabilitation and/or post-operative activities, or also during sports activities, to exert controlled pressure on the rear part of the calf, compensating for the dysfunction of a partially or totally impaired or reconstructed ligament.

This invention substantially concerns a structured knee brace equipped with mechanisms that provide appropriate support to the femoral-tibial joint with respect to its posterior movement, thus helping to make up for the market lack of suitable braces for this specific need.

This invention can be applied in the orthopedics sector with particular reference to the manufacturers of prostheses and braces.

### BACKGROUND ART

It is known that the posterior cruciate ligament (hereinafter PCL) is the anatomical structure designed to act as the primary control of the posterior movement of the tibia with respect to the femur to all the flexion angles of the knee joint.

Injury of the posterior cruciate ligament is a condition which occurs much less frequently than with its more know counterpart which is the anterior cruciate ligament, taking into account that both these ligaments contribute together to making the knee stable.

Ligaments are bands of highly resistant tissue that hold the bones together. The cruciate ligaments of the knee connect the femur to the tibia, in such a way that the posterior cruciate ligament and the anterior cruciate ligament form an "X" at the centre of the knee.

If one of these knee ligaments is injured, it can cause pain, swelling and a sensation of instability of the knee. The rupture of one or more of these ligaments, or trauma that causes them to become slack lead to a loss of balance between the mobility of the articular components of the knee and the stability of the joint in question, triggering "abnormal" kinematic patterns and damage to the tissues surrounding the joint.

According to the literature, from 5% to 20% of injuries to ligaments of the knee and more than 60% of those treated in emergency departments involve the PCL.

Treatment of injuries to the PCL can be surgical or conservative: in both cases the protocol foresees a period in which the patient must protect the joint while guaranteeing appropriate support for posterior movement.

According to the known technique for dealing with problems related to the posterior cruciate ligament, partial or total plaster casts are used, or generic knee braces which guarantee a certain overall stability of the knee regardless of the specific problems of the two ligaments, these solutions thus being inadequate for the specific requirements.

Document EP 1302184 A1 discloses a knee brace according to the preamble of claim 1. Document US-A-4361142 discloses a an orthotic joint and a knee orthosis for the protective treatment of ligamentous injuries. The joint includes coacting guide members that are both slidable and pivotable with respect to each other and, when assembled and supported in pairs on opposite sides of a patient's knee, allow freedom of movement of the knee except for those movements that would normally be controlled by the ligaments requiring protection. An arrangement of straps connect each pair of guide members and are tensioned (and untensioned) in sequence to perform operations in conjunction with the articulating surfaces of the members that would otherwise be demanded from the protected ligaments.

### DESCRIPTION OF THE INVENTION

This invention proposes to provide a functional knee brace for PCL support, which is able to overcome the lack of specific knee braces for the posterior cruciate ligament.

The invention also proposes to provide a knee brace designed to support the posterior cruciate ligament, which can be used for rehabilitation and/or post-operative activities or also as an aid during sports activities.

This is achieved by means of a functional knee brace designed to support the posterior cruciate ligament, whose features are described in the main claim.

The dependent claims describe advantageous embodiments of the invention.

The main advantages of this solution concern first of all the fact that a specific knee brace is provided which can be used by patients with PCL injuries.

More specifically, this invention concerns a structured knee brace equipped with mechanisms that provide appropriate support to the femoral-tibia joint with respect to its posterior movement.

From a construction point of view, as will be better seen below, the general architecture of the knee brace according to the invention is that of a functional prosthesis characterised by a lower arch which is longer than "conventional" types and which also presents an adjustable posterior thrust mechanism which is fixed to the rear part of the lower arch so that it acts on the calf in a posterior-anterior direction.

### DESCRIPTION OF THE DRAWINGS

Other features and advantages of the invention will become evident on reading the description given below of one embodiment of the invention, provided as a non-binding example, with the help of the accompanying drawings in which:

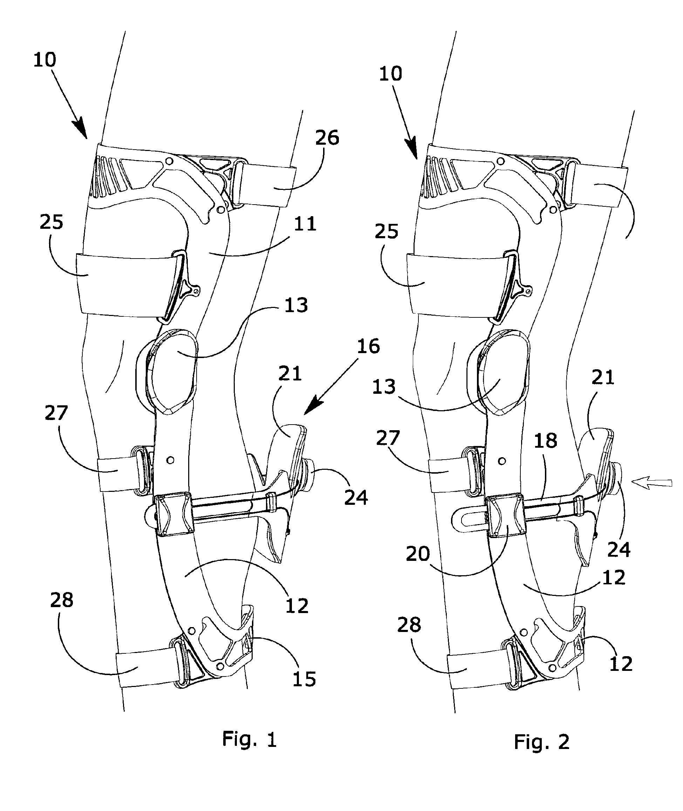

- figure 1 represents an overall view of the PCL support knee brace according to the invention, fitted close to the knee and with the thrust mechanism loosened;
- figure 2 represents the same overall view of the PCL support knee brace according to the invention, fitted close to the knee and with the thrust mechanism tightened;
- figures 3 and 4 show schematic and prospective views of the knee brace according to the invention from two different angles;
- figure 5 is a schematic view showing a detail of the posterior thrust mechanism;
- figure 6 is an anatomical view showing the position of the posterior cruciate ligament with respect to the knee.

### DESCRIPTION OF ONE EMBODIMENT OF THE INVENTION

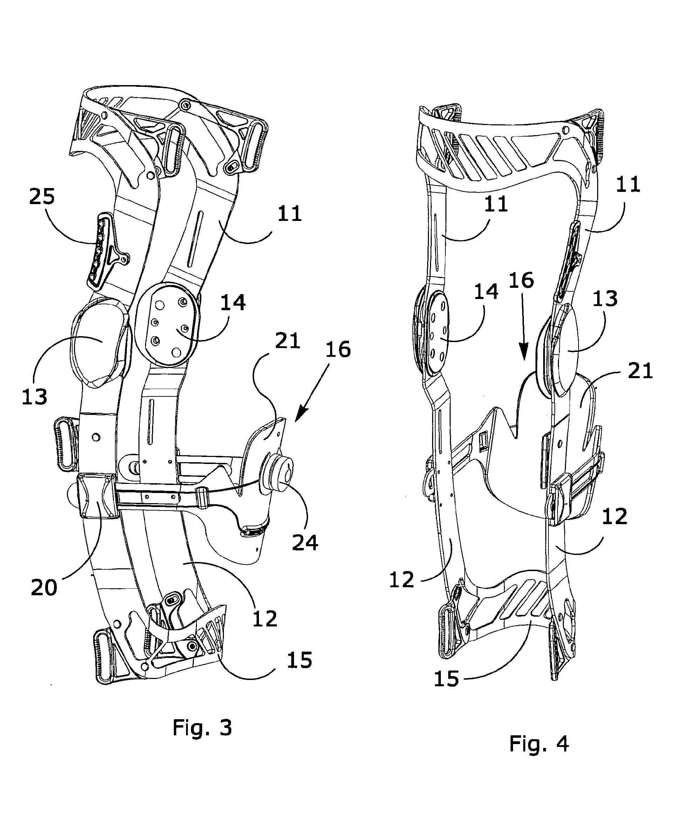

With reference to the accompanying drawings, and initially to figures 3 and 4, the functional knee brace according to the invention designed to support the posterior cruciate ligament, indicated globally with reference number 10, is a structured knee brace equipped with mechanisms that provide appropriate support to the femoral-tibial joint with respect to its posterior movement.

The knee brace 10 basically consists of two parts 11 and 12 joined together by traditional type articulated mechanisms 13 and 14, designed to be respectively positioned in the femoral area and the tibial area of the leg for the treatment of PCL injuries.

The structure of the knee brace according to the invention is that of a functional prosthesis, and the lower tibial sector 12, with a backward-facing positioning curvature 15, consists of an arch which is longer than that of "conventional" knee braces.

In particular, the lower posterior support 15 of the frame must be positioned below the calf muscles in order to obtain a bigger lever arm with respect to the traditional configuration.

According to the invention, the lower part 12 of the knee brace comprises a posterior thrust mechanism 16 which is fixed to the lower arch so that it acts on the calf in a posterior - anterior direction.

This thrust mechanism consists of an arched frame provided with two arms 17 and 18 attached to respective blocks 19 and 20 fixed at the two opposite sides of the lower part 12 of the knee brace in such a way that they can slide in a horizontal direction.

The central part of the thrust mechanism 16 comprises a pad 21, the inner part of which is designed to rest against and compress the calf.

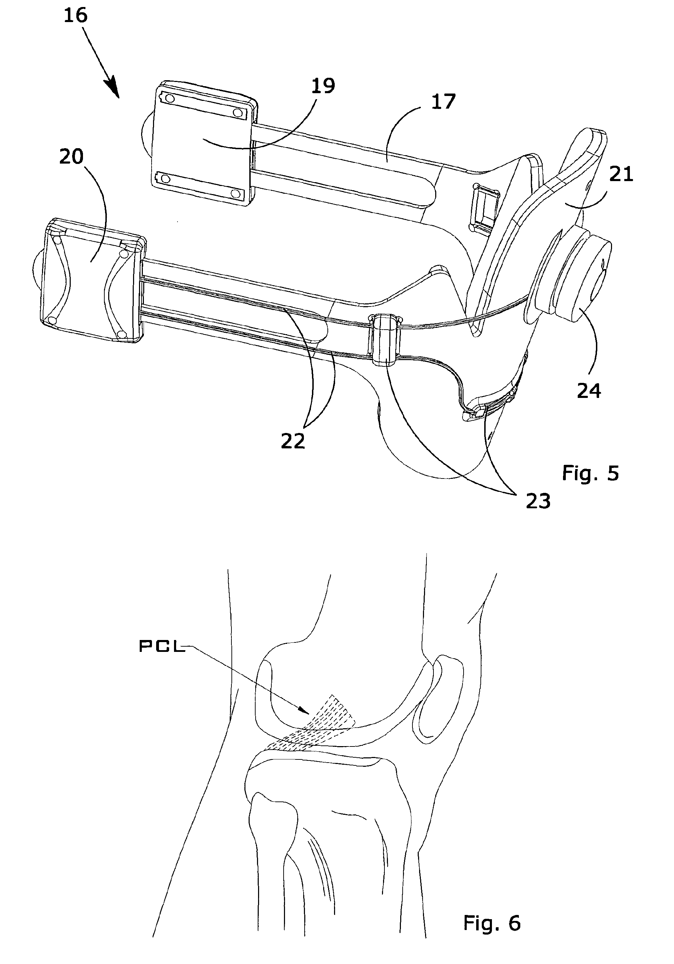

The pad 21 compresses the calf area by means of a tightening mechanism visible in detail in figure 5, which in this example consists of a cable 22 that, passing through appropriate gears 23 situated inside the blocks 19 and 20 and being intercepted by an adjustable tightening knob 24, makes it possible to move the pad 12 towards the calf and compress it.

The knee brace according to the invention also presents a front strap 25 positioned on the upper arch 11 of the brace. This strap 25 is larger than traditional straps in order to guarantee a larger gripping surface with the dual aim of increasing the stability of the brace and the level of comfort for the user.

Additional upper/rear 26 and lower/front straps 27 and 28 ensure perfect blocking of the brace.

The use of this brace therefore involves it being worn in such a way that the backward-facing positioning curve 15 of the lower arch is placed below the calf muscles, with the pad 21 close to the calf.

The brace is then fixed in place by means of the straps 25, 26, 27 and 28 and the pad 21 is compressed against the calf by turning the adjustment knob 24, until the required pressure is achieved.

According to other embodiments of the invention, the tightening knob 24 can be replaced by any other device designed for the same purpose, such as ratchet or adjustable hooking devices or the like.

The invention is described above with reference to a preferred embodiment. It is nevertheless clear that the invention is susceptible to numerous variations which lie within the scope of its disclosure, within the framework of technical equivalents.

## Claims

1. A functional knee brace designed to support the posterior cruciate ligament and the femoral-tibial joint in its posterior movement, said brace consisting of two parts (11, 12) joined to each other by articulated mechanisms (13, 14) to be respectively positioned in the femoral and tibial parts of the leg, wherein the lower tibial sector (12) presents a backward-facing positioning curve (15) with a longer arch in order to be positioned below the calf muscles, **characterised in that** the lower part (12) of the knee brace comprises a posterior thrust mechanism (16) which is fixed to the lower arch in such a way as to exert pressure on the calf in a posterior-anterior direction, whereby said thrust mechanism consists of an arched frame provided with two arms (17, 18) attached to respective blocks (19, 20) fixed to the two opposite sides of the lower part (12) of the knee brace in such a way that the arms can slide in a horizontal direction.

2. A knee brace according to claim 1, **characterised in that** the central part of the thrust mechanism (16) comprises a pad (21), the inner part of which is designed to rest against and compress the calf.

3. A knee brace according to any one of the preceding claims, **characterised in that** the pad (21) compresses the calf by means of a tightening mechanism.

4. A knee brace according to any one of the preceding claims, **characterised in that** the tightening device consists of a cable (22) which, passing through appropriate gears (23) positioned inside the blocks (19, 20) and being intercepted by an adjustable tightening knob (24), makes it possible to move the pad (12) towards the calf and compress it.

5. A knee brace according to any one of the preceding claims, **characterised in that** the knee brace presents a front strap (25) positioned on the upper arch (11) of the brace, this strap (25) being larger than traditional straps to ensure a larger gripping surface to increase the stability of the brace and the level of comfort for the user.

6. A knee brace according to any one of the preceding claims, **characterised by** the presence of additional upper/posterior (26) and lower/anterior straps (27, 28), which ensure perfect blocking of the brace.

7. A knee brace according to any one of the preceding claims, **characterised in that** the tightening device can be any type designed for the same purpose, such as ratchet or adjustable hooking device or the like.

## Patentansprüche

1. Funktionelle Knieschiene, die zum Stützen des hinteren Kreuzbandes und des Kniegelenks in seiner posterioren Bewegung konstruiert ist, wobei die Schiene aus zwei Teilstücken (11, 12) besteht, die miteinander durch Gelenkmechanismen (13, 14) verbunden sind, um entsprechend in den Oberschenkel- und Schienbeinbereichen des Beines positioniert zu werden, wobei der untere Schienbeinabschnitt (12) einen rückwärts gerichteten Positionierungsbogen (15) mit einem längeren Bogenstück aufweist, um unterhalb der Wadenmuskeln positioniert zu werden, **dadurch gekennzeichnet, dass** das untere Teilstück (12) der Knieschiene eine hintere Schiebevorrichtung (16) umfasst, die an dem unteren Bogenstück derart befestigt ist, dass sie auf die Wade in einer posterioren-anterioren Richtung Druck ausübt, wobei die Schiebevorrichtung aus einem bogenförmigen Rahmen besteht, der mit zwei Armen (17, 18) versehen ist, die an entsprechenden Blöcken (19, 20) angebracht sind, welche an den beiden gegenüberliegenden Seiten des unteren Teilstücks (12) der Knieschiene derart befestigt sind, dass die Arme in einer horizontalen Richtung gleiten können.

2. Knieschiene nach Anspruch 1, **dadurch gekennzeichnet, dass** das Mittelteil der Schiebevorrichtung (16) ein Polsterelement (21) aufweist, dessen Innenteil konstruiert ist, an der Wade anzuliegen und diese zusammenzudrücken.

3. Knieschiene nach einem der vorhergehenden Ansprüche, **dadurch gekennzeichnet, dass** das Polsterelement (21) die Wade mit Hilfe einer Festziehvorrichtung zusammendrückt.

4. Knieschiene nach einem der vorhergehenden Ansprüche, **dadurch gekennzeichnet, dass** die Festziehvorrichtung aus einem Drahtseil (22), das durch die entsprechenden zwischen den Blöcken (19, 20) positionierten Eingriffelemente (23) hindurchgeht und durch einen verstellbaren Festziehknopf (24) unterbrochen wird, besteht, welches es ermöglicht, das Polsterelement (12) in Richtung der Wade zu bewegen und sie zusammenzudrücken.

5. Knieschiene nach einem der vorhergehenden Ansprüche, **dadurch gekennzeichnet, dass** die Knieschiene ein vorderes Halteband (25) aufweist, das an dem oberen Bogen (11) der Schiene positioniert ist, wobei dieses Halteband (25) größer als herkömmliche Haltebänder ist, damit eine größere Greiffläche gewährleistet ist, um die Stabilität der Schiene und das Komfortniveau für den Benutzer zu erhöhen.

6. Knieschiene nach einem der vorhergehenden Ansprüche, **gekennzeichnet durch** das Vorhandensein von zusätzlichen oberen/posterioren (26) und unteren/anterioren Haltebändern (27, 28), welche die vollständige Arretierung der Schiene gewährleisten.

7. Knieschiene nach einem der vorhergehenden Ansprüche, **dadurch gekennzeichnet, dass** die Festziehvorrichtung jede Art sein kann, die für denselben Zweck konstruiert ist, wie etwa eine Ratsche oder eine verstellbare Einhakvorrichtung oder dergleichen.

## Revendications

1. Genouillère fonctionnelle conçue pour maintenir le ligament croisé postérieur et l'articulation fémoro-tibiale lors de son mouvement postérieur, ladite genouillère étant formée de deux parties (11, 12) reliées entre elles par des mécanismes articulés (13, 14) et destinées à être positionnées respectivement dans les parties fémorale et tibiale de la jambe, la section tibiale inférieure (12) présentant une courbe de positionnement qui est dirigée vers l'arrière (15) et qui possède un arceau plus long pour le positionnement au-dessous des muscles du mollet, **caractérisée en ce que** la partie inférieure (12) de la genouillère comprend un mécanisme de poussée postérieur (16) qui est fixé à l'arcade inférieure de manière à exercer une pression sur le mollet dans une direction postéro-antérieure, ce pour quoi ledit mécanisme de poussée est formé d'un cadre en arceau muni de deux bras (17, 18) attachés à des blocs respectifs (19, 20) fixés aux deux côtés opposés de la partie inférieure (12) de la genouillère de sorte que les bras peuvent coulisser dans une direction horizontale.

2. Genouillère selon la revendication 1, **caractérisée en ce que** la partie centrale du mécanisme de poussée (16) comporte un coussinet (21) dont la partie intérieure est conçue pour reposer contre le mollet et le comprimer.

3. Genouillère selon l'une quelconque des revendications précédentes, **caractérisée en ce que** le coussinet (21) comprime le mollet au moyen d'un mécanisme de serrage.

4. Genouillère selon l'une quelconque des revendications précédentes, **caractérisée en ce que** le dispositif de serrage est formé d'un câble (22) qui, en passant par des engrenages appropriés (23) positionnés à l'intérieur des blocs (19, 20) et étant intercepté par au bouton de serrage réglable (24), permet de déplacer le coussinet (12) en direction du mollet et de le comprimer.

5. Genouillère selon l'une quelconque des revendications précédentes, **caractérisée en ce que** la genouillère comporte une sangle avant (25) située sur l'arcade supérieure (11) de la genouillère, cette sangle (25) étant plus grande que les sangles traditionnelles afin d'assurer une plus grande surface de préhension pour augmenter la stabilité de le genouillère et le niveau de confort de l'utilisateur.

6. Genouillère selon l'une quelconque des revendications précédente, **caractérisée par** la présence de sangles postéro-supérieures supplémentaires (26) et des sangles antéro-inférieures supplémentaires (27, 26), qui assurent le blocage parfait de la genouillère.

7. Genouillère selon l'une quelconque des revendications précédentes, **caractérisée en ce que** le dispositif de serrage peut être de n'importe quel type conçu dans le même but, tel qu'un cliquet ou un dispositif d'accrochage réglable ou similaire.
